(19) Europäisches Patentamt
European Patent Office
Office européen des brevets

(11) **EP 4 085 828 A1**

(12) **EUROPEAN PATENT APPLICATION**
published in accordance with Art. 153(4) EPC

(43) Date of publication:
**09.11.2022 Bulletin 2022/45**

(51) International Patent Classification (IPC):
**A61B 5/053** (2021.01)      **A61B 5/026** (2006.01)

(21) Application number: **20909510.8**

(22) Date of filing: **04.08.2020**

(86) International application number:
**PCT/CN2020/106754**

(87) International publication number:
**WO 2021/135211 (08.07.2021 Gazette 2021/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.12.2019 CN 201911424085**

(71) Applicant: **Beijing Huarui Boshi Medical Imaging Technology Co., Ltd.**
**Beijing 102609 (CN)**

(72) Inventors:
• **ZHANG, Ke**
  **Beijing 102609 (CN)**
• **ZHANG, Xin**
  **Beijing 102609 (CN)**
• **WANG, Yibing**
  **Beijing 102609 (CN)**
• **YU, Yang**
  **Beijing 102609 (CN)**

(74) Representative: **Dr. Weitzel & Partner Patent- und Rechtsanwälte mbB Friedenstrasse 10 89522 Heidenheim (DE)**

(54) **ELECTRICAL IMPEDANCE TOMOGRAPHY BASED METHOD AND DEVICE FOR GENERATING THREE-DIMENSIONAL BLOOD PERFUSION IMAGE**

(57)      Provided in the present disclosure are an electrical impedance tomography based method and device for generating a three-dimensional blood perfusion image. The method (100) comprises: using an electrode array distributed in a three-dimensional space to perform electrical impedance measurement on a human body region to be measured so as to obtain an electrical impedance measurement signal (110); and on the basis of a blood perfusion signal in the electrical impedance measurement signal, reconstructing a three-dimensional blood perfusion image by means of an image reconstruction algorithm (120). Therefore, a three-dimensional image of electrical impedance variations caused by blood perfusion can be generated; and compared with a two-dimensional image in the prior art, the three-dimensional image can more intuitively reflect the blood perfusion condition of a volume area in the three-dimensional space of a human body region, and facilitates image analysis and comparison and disease detection and diagnosis.

100

110
Performing, by using an electrode array distributed in a three-dimensional space, electrical impedance measurement on a human body region to be measured so as to obtain an electrical impedance measurement signal

120
Reconstructing, on the basis of a blood perfusion signal in the electrical impedance measurement signal, a three-dimensional blood perfusion image by means of an image reconstruction algorithm

FIG. 1

EP 4 085 828 A1

## Description

The present disclosure claims the priority to Chinese Patent Application No. CN201911424085.5, titled "ELECTRICAL IMPEDANCE TOMOGRAPHY BASED METHOD AND DEVICE FOR GENERATING THREE-DIMENSIONAL BLOOD PERFUSION IMAGE", filed on December 31, 2019, which is incorporated herein by reference in its entirety.

## TECHNICAL FIELD

The present disclosure relates to the technology of electrical impedance tomography, and in particular to an electrical impedance tomography based method and device for generating three-dimensional blood perfusion image.

## BACKGROUND

Electrical impedance tomography (EIT) is a non-invasive technique that aims at the resistivity distribution in the human body or other organisms and reconstructs images of tissues in vivo. The human body is a large biological electrical conductor, and all tissues and organs have certain impedance. When a disease occurs in a certain part of an organ of the human body, the impedance of the diseased part must be different from that of other parts. Therefore, impedance measurement can be used to diagnose the diseases of human organs.

The existing EIT methods can only reconstruct two-dimensional blood perfusion images. The two-dimensional image reflects an electrical impedance change caused by blood perfusion in a certain section of a human body region to be measured, but it is hard to reflect blood perfusion in a volume area in a three-dimensional space.

Therefore, it is necessary to provide an electrical impedance tomography based method and device for generating three-dimensional blood perfusion image, which are able to reconstruct a three-dimensional blood perfusion image based on a result of an electrical impedance measurement.

## SUMMARY

As stated above, in order to solve the problems existing in the prior art, an objective of the present disclosure is to provide a method and device for generating a three-dimensional blood perfusion image. The method and device perform electrical impedance measurement on a human body region to be measured, extract a blood perfusion signal from a measurement signal, and reconstruct a three-dimensional blood perfusion image. Alternatively, the method and device reconstruct a three-dimensional differential image based on the measurement signal, and extract the three-dimensional blood perfusion image reflected by the blood perfusion signal in the measurement signal from the three-dimensional differential image. The three-dimensional blood perfusion image generated by the method and device can be displayed by a display apparatus.

According to an embodiment of the present disclosure, a first aspect of the present disclosure provides an electrical impedance tomography based method for generating three-dimensional blood perfusion image. The method may include the following steps: performing, by using an electrode array distributed in a three-dimensional space, electrical impedance measurement on a human body region to be measured so as to obtain an electrical impedance measurement signal; and reconstructing, on the basis of a blood perfusion signal in the electrical impedance measurement signal, a three-dimensional blood perfusion image by means of an image reconstruction algorithm.

In an implementation, the method according to the first aspect of the present disclosure may further include: extracting the blood perfusion signal from the electrical impedance measurement signal; and reconstructing, by using the extracted blood perfusion signal, the three-dimensional blood perfusion image by means of the image reconstruction algorithm.

In this case, the extracting the blood perfusion signal from the electrical impedance measurement signal may further include: extracting the blood perfusion signal from the electrical impedance measurement signal by using a time-frequency characteristic thereof.

In an implementation, the extracting the blood perfusion signal from the electrical impedance measurement signal by using a time-frequency characteristic thereof may include: separating, by a band-pass filter, a signal of a specific frequency range from the electrical impedance measurement signal to form the blood perfusion signal.

In addition, the method according to the first aspect of the present disclosure may further include: reconstructing, on the basis of the electrical impedance measurement signal, a three-dimensional differential image by means of the image reconstruction algorithm; and extracting the three-dimensional blood perfusion image reflected by the blood perfusion signal in the electrical impedance measurement signal from the three-dimensional differential image.

In this case, the extracting the three-dimensional blood perfusion image reflected by the blood perfusion signal in the electrical impedance measurement signal from the three-dimensional differential image may further include: extracting the three-dimensional blood perfusion image by using a time-frequency characteristic of a pixel in the three-dimensional differential image.

In an implementation, the extracting the three-dimensional blood perfusion image by using a time-frequency characteristic of a pixel in the three-dimensional differential image may include: separating, by a band-pass filter, a signal of a specific frequency range from a time-domain signal of each pixel in the three-dimensional differential image, so as to form a time-domain signal of a corresponding pixel in the three-dimensional blood perfusion image.

In the method according to the first aspect of the present disclosure, in an implementation, the electrode array may be disposed on one or more impedance belts, an electrode vest, or an electrode cap, so as to realize three-dimensional distribution of electrodes.

According to an embodiment of the present disclosure, a second aspect of the present disclosure provides an electrical impedance tomography based device for generating a three-dimensional blood perfusion image. The device may include: an electrode array, distributed in a three-dimensional space, and configured to perform electrical impedance measurement on a human body region to be measured so as to obtain an electrical impedance measurement signal; and an image reconstruction processor, configured to execute a program stored in a memory, so as to reconstruct, on the basis of a blood perfusion signal in the electrical impedance measurement signal, a three-dimensional blood perfusion image by means of an image reconstruction algorithm.

In the device according to the second aspect of the present disclosure, in an implementation, the image reconstruction processor may be further configured to: execute a program stored in the memory, so as to extract the blood perfusion signal from the electrical impedance measurement signal; and reconstruct, by using the extracted blood perfusion signal, the three-dimensional blood perfusion image by means of the image reconstruction algorithm.

In addition, in the device according to the second aspect of the present disclosure, the image reconstruction processor may be further configured to: execute a program stored in the memory, so as to reconstruct a three-dimensional differential image by means of the image reconstruction algorithm; and extract the three-dimensional blood perfusion image reflected by the blood perfusion signal in the electrical impedance measurement signal from the three-dimensional differential image.

In the device according to the second aspect of the present disclosure, in an implementation, the electrode array may be disposed on one or more impedance belts, an electrode vest, or an electrode cap, so as to realize three-dimensional distribution of electrodes.

A three-dimensional image of electrical impedance variations caused by blood perfusion can be generated by the method and device for generating a three-dimensional blood perfusion image according to the embodiments of the present disclosure. Compared with two-dimensional images in the prior art, the three-dimensional image can more intuitively reflect the blood perfusion condition of a volume area in the three-dimensional space of a human body region, and facilitates image analysis and comparison, and disease detection and diagnosis.

## BRIEF DESCRIPTION OF THE DRAWINGS

The present disclosure is described below with reference to the accompanying drawings and embodiments.

FIG. 1 is a schematic flowchart of an electrical impedance tomography based method for generating a three-dimensional blood perfusion image according to an embodiment of the present disclosure;
FIGS. 1A and 1B are schematic flowcharts of the electrical impedance tomography based method for generating a three-dimensional blood perfusion image according to two preferred embodiments of the present disclosure;
FIG. 2 illustrates an example of measurement data of a human thoracic cavity according to the preferred embodiment of FIG. 1A, where (a) shows a time-domain signal, and (b) shows a frequency-domain signal;
FIG. 3 illustrates perfusion-related signals extracted by filtering according to the preferred embodiment of FIG. 1A, where (a) shows a time-domain signal, and (b) shows a frequency-domain signal;
FIG. 4 illustrates examples of a three-dimensional differential image and a time-frequency signal of a sample pixel point according to the preferred embodiment of FIG. 1B, where (a) shows the three-dimensional differential image, (b) shows a time-domain signal of the sample pixel, and (c) shows a frequency-domain signal of the sample pixel;
FIG. 5 illustrates perfusion-related signals of the sample pixel extracted by filtering according to the preferred embodiment of FIG. 1B, where (a) shows a time-domain signal, and (b) shows a frequency-domain signal;
FIG. 6 shows a three-dimensional blood perfusion image of a human lung according to a preferred embodiment of the present disclosure; and
FIG. 7 is a schematic block diagram of an electrical impedance tomography based device for generating a three-dimensional blood perfusion image according to an embodiment of the present disclosure.

## DETAILED DESCRIPTION OF THE EMBODIMENTS

The accompanying drawings are for illustrative purposes only and should not be construed as limiting the present disclosure. The technical solutions of the present disclosure are described in further detail below with reference to the accompanying drawings and embodiments.

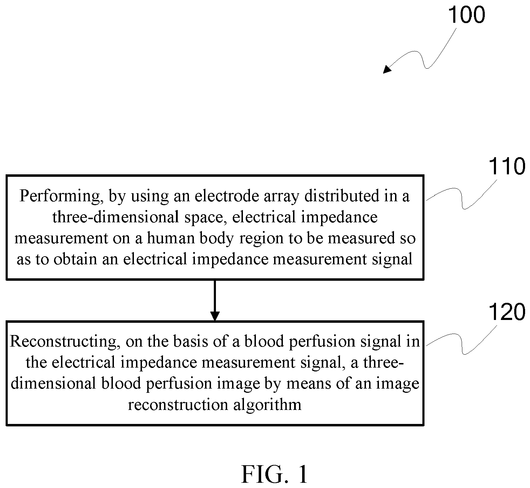

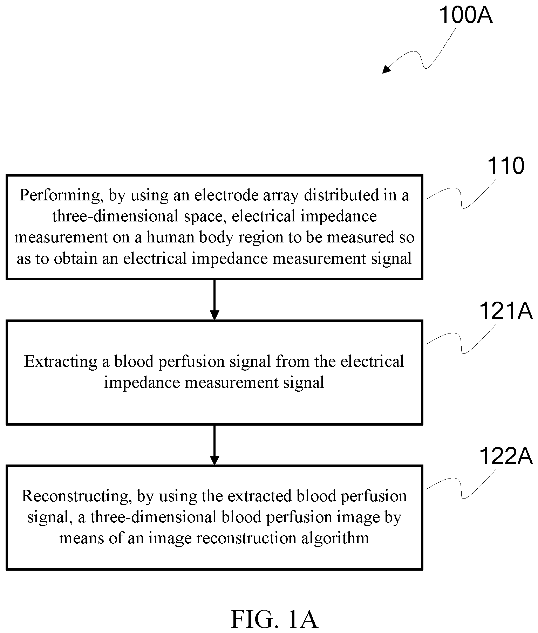

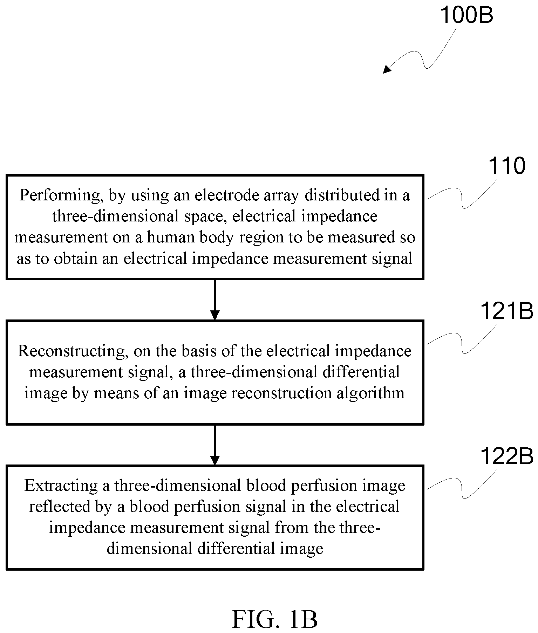

FIG. 1 is a schematic flowchart of an electrical impedance tomography based method 100 for generating a three-dimensional blood perfusion image according to an embodiment of the present disclosure.

As shown in FIG. 1, the method 100 begins from Step 110, where an electrical impedance signal of a human body is measured. In an implementation, the electrical impedance measurement is performed on a human body region to be measured by using an electrode array distributed in a three-dimensional space so as to obtain an electrical impedance measurement signal.

During the electrical impedance measurement, firstly the electrode array should be fixed around the human body region to be measured. The electrode array includes multiple electrodes distributed in the three-dimensional space. Then, the human body region to be measured is excited by the electrode array, and a resulting response is measured. For example, current excitations are applied to electrodes in turn, and resulting voltage signals are measured on other electrodes in turn.

In an implementation, a sensing module including the electrodes is fixed in the human body region to be measured, such as around the thoracic cavity, brain, abdomen or limbs, in the form of an electrode array on an impedance belt, an electrode vest or an electrode cap. In some embodiments, the electrodes may take the form of in-vivo electrodes. The so-called internal electrode refers to placing the electrodes in the esophagus, trachea or other internal positions in the human body.

According to a preferred embodiment of the present disclosure, the signal measurement may be performed by an electrode array in the form of one or more impedance belts, an electrode vest, or an electrode cap, etc. That is, the electrode array is disposed on one or more impedance belts, an electrode vest, or an electrode cap, so as to realize three-dimensional distribution of the electrodes. In an implementation, in order to make the reconstructed image have three-dimensional resolution, the electrode array is generally distributed in a three-dimensional space, rather than in a two-dimensional plane or an approximate two-dimensional plane. In order to distribute the electrode array in a three-dimensional space, multiple impedance belts may be used. Alternatively, an electrode vest or an electrode cap, in which the electrodes are distributed in three dimensions, may also be used.

The electrical impedance measurement signal may be a voltage signal, specifically a complex voltage signal. The complex voltage signal may be expressed in terms of amplitude and phase, or it may be expressed in terms of real and imaginary parts.

Then, the method proceeds to Step 120, where a three-dimensional blood perfusion image is reconstructed by means of an image reconstruction algorithm based on a blood perfusion signal in the electrical impedance measurement signal.

Step 120 may be implemented in two ways. FIGS. 1A and 1B are flowcharts of the electrical impedance tomography based method for generating a three-dimensional blood perfusion image according to two preferred embodiments 100A and 100B of the present disclosure.

As shown in FIG. 1A, according to the preferred embodiment 100A of the present disclosure, after Step 110 and in Step 121A, the blood perfusion signal is extracted from the electrical impedance measurement signal.

In this step, it is necessary to extract the blood perfusion signal from the electrical impedance measurement signal acquired in the previous step. According to a preferred embodiment of the present disclosure, the blood perfusion signal may be extracted from the electrical impedance measurement signal acquired in the previous step by using a time-frequency characteristic thereof. In an implementation, the blood perfusion signal is separated from the measured electrical impedance signal by a filter.

The following takes a measurement signal of a human thoracic cavity as an example to illustrate this step.

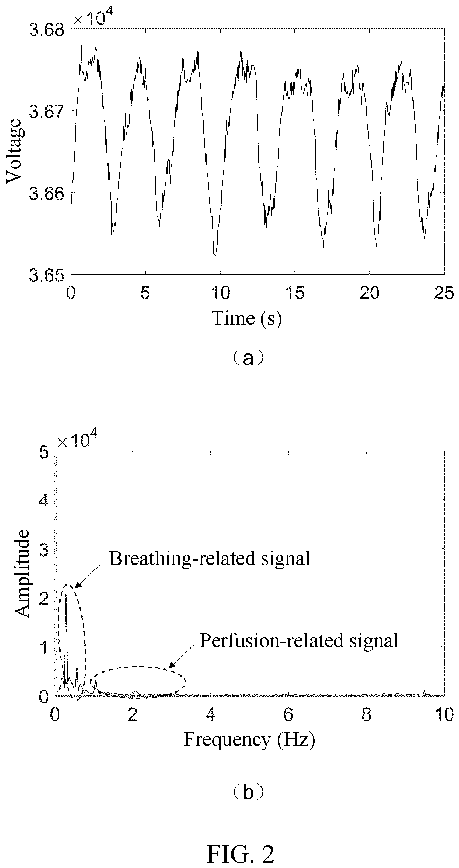

FIG. 2 illustrates an example of measurement data of the human thoracic cavity according to the preferred embodiment of FIG. 1A, and FIG. 2(a) shows a time-domain graph of a certain measurement data. The curve in the figure denotes a voltage signal measured from a specific electrode when the specific electrode is excited. Similar data were acquired in other excitation-measurement situations. It should be noted that the ordinate value in the figure represents a value directly read from a digital voltmeter, which has not yet been converted into a voltage value. FIG. 2(b) shows a frequency-domain graph of the measured signal. In an implementation, it may be acquired by Fourier transform of the signal in FIG. 2(a). A breathing-related signal component and a perfusion-related signal component can be distinguished from FIG. 2(b). To extract the perfusion-related signal component, a band-pass filter may be designed.

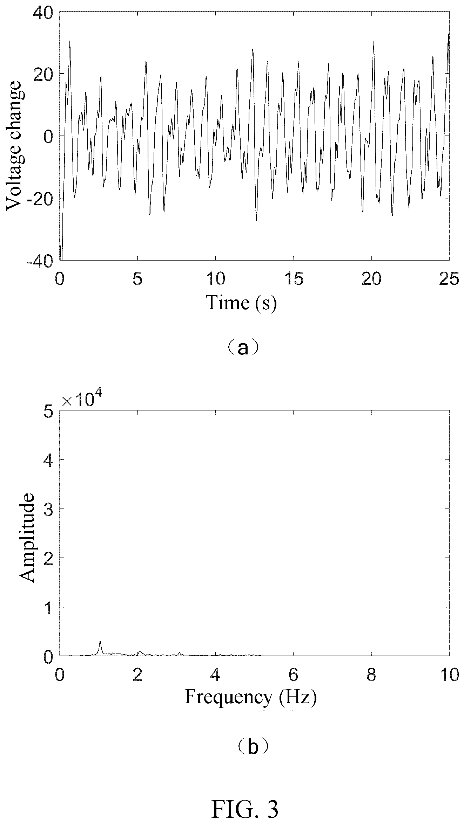

The filtered signal is shown in FIG. 3. FIG. 3 illustrates the perfusion-related signal extracted by filtering according to the preferred embodiment of FIG. 1A. FIG. 3(a) shows a time-domain graph of the filtered signal, and FIG. 3(b) shows a frequency-domain graph of the filtered signal.

In the above example, a signal of a specific frequency range is separated from the electrical impedance measurement signal by a band-pass filter to form the blood perfusion signal.

In Step 122A, based on the extracted blood perfusion signal, the three-dimensional blood perfusion image is reconstructed by the image reconstruction algorithm.

Generally, if the reconstruction of a pulmonary blood perfusion image is taken as an example, the reconstruction process is as follows: first, extracting perfusion signals from measurement data, and then performing image reconstruction based on a difference of the perfusion signals at different times. The three-dimensional blood perfusion image reflects changes in electrical impedance, such as changes in electrical conductivity, in the measured human body region due to blood perfusion. Therefore, the changes of lung blood content at different times are displayed accordingly.

In a preferred embodiment of the present disclosure, the image reconstruction algorithm is a linear differential imaging algorithm. The following is an example of a linear differential imaging algorithm.

It is assumed that a time-domain form of the perfusion signal extracted in the previous step is u(t), t being a time variable. Thus, the EIT differential reconstruction can be expressed as the following least squares problem:

$$\min_{\delta\sigma} \|J \cdot \delta\sigma - \delta u\|^2 + \alpha \|R \cdot \delta\sigma\|^2$$

wherein J denotes a Jacobian matrix; $\delta u = u(t_2) - u(t_1)$ denotes a change of the signal at a time $t_2$ relative to a time $t_1$; $\delta\sigma$ denotes a conductivity change caused by blood perfusion at the two times; R denotes a regularization matrix; and $\alpha$ denotes a regularization parameter. $\delta\sigma$ is defined in a discretized three-dimensional model, such as a tetrahedral grid or a voxel grid. The solution to the above problem is

$$\delta\sigma_* = (J^T \cdot J + \alpha R^T \cdot R)^{-1} \cdot J^T \cdot \delta u.$$

Supposing that $D = (J^T \cdot J + \alpha R^T \cdot R)^{-1} \cdot J^T$, then the above expression can be rewritten as:

$$\delta\sigma_* = D \cdot \delta u.$$

$\delta\sigma_*$ is the calculated blood perfusion image.

In the above example, the linear differential imaging algorithm is specifically used to calculate and reconstruct the three-dimensional blood perfusion image. However, those of ordinary skill in the art should understand that the image reconstruction algorithms that can be utilized in the present disclosure may include a variety of image reconstruction algorithms: linear or non-linear, iterative or non-iterative, random or deterministic image reconstruction algorithms, etc.

Then, refer to FIG. 1 and FIG. 1B. As shown in FIG. 1B, Step 120 shown in FIG. 1 further includes another implementation 100B. That is, Step 121B, i.e., reconstructing a three-dimensional differential image by means of an image reconstruction algorithm based on the electrical impedance measurement signal, is performed after Step 110 shown in FIG. 1.

In this step, the image reconstruction algorithm may be the same image reconstruction algorithm as in the first preferred embodiment 100A of the present disclosure.

Then proceeds to Step 122B, where the three-dimensional blood perfusion image reflected by the blood perfusion signal in the electrical impedance measurement signal is extracted from the three-dimensional differential image. According to a preferred embodiment of the present disclosure, the three-dimensional blood perfusion image may be extracted from the three-dimensional differential image by using a time-frequency characteristic of the image signal. In an implementation, the three-dimensional blood perfusion image is separated from the three-dimensional differential image by a filter.

The following is an example of the above measurement signal of the human thoracic cavity.

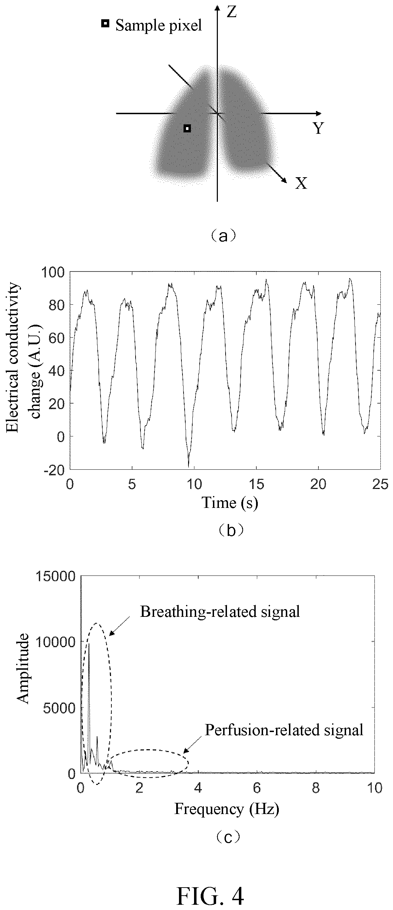

FIG. 4 illustrates a reconstructed three-dimensional differential image according to the preferred embodiment of FIG. 1B. FIG. 4(a) shows an example of the three-dimensional differential image, FIG. 4(b) shows a time-domain signal of a sample pixel selected from the three-dimensional differential image, and FIG. 4(c) shows a frequency-domain signal of the sample pixel. In an implementation, the signal shown in FIG. 4(c) may be acquired by Fourier transform of the signal shown in FIG. 4(b). A breathing-related signal component and a perfusion-related signal component can be distinguished from FIG. 4(c). In order to extract the perfusion-related signal component, a band-pass filter may be designed.

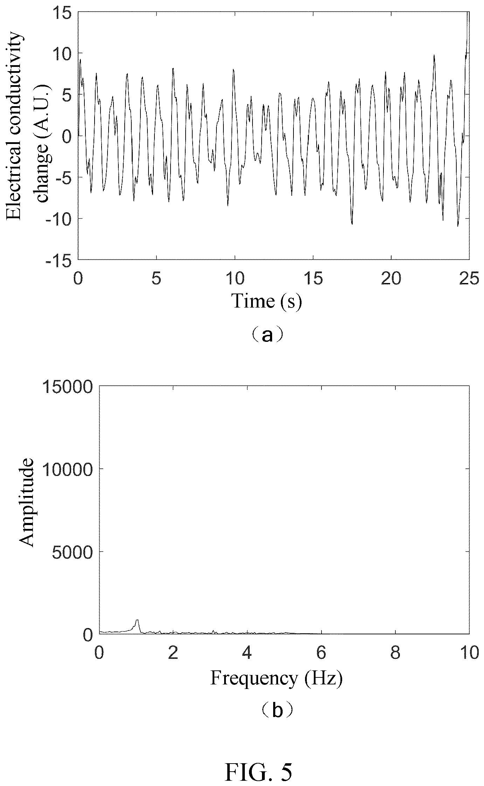

The band-pass filter performs a filtering operation on the time-domain signal of each pixel in the three-dimensional differential image. The signal acquired by filtering the sample pixel is shown in FIG. 5, wherein FIG. 5(a) shows a filtered time-domain signal, and FIG. 5(b) shows a filtered frequency-domain signal.

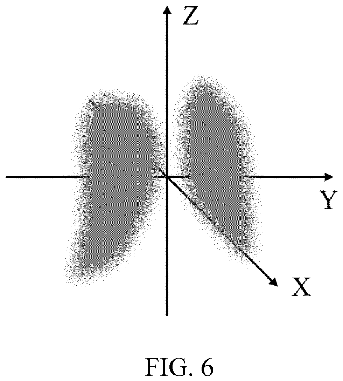

A three-dimensional blood perfusion image is acquired after filtering each pixel in the above three-dimensional differential image. The three-dimensional blood perfusion image reflects changes in electrical impedance, such as changes in electrical conductivity, in the measured human body region due to blood perfusion. Therefore, the image reflects the changes in lung blood content at different times. FIG. 6 shows a schematic diagram of a three-dimensional blood perfusion image of a human lung generated by the method as described above including embodiment 100A and embodiment 100B.

It is apparent that the difference between the two implementations of Step 120 of the method 100 shown in FIG. 1 lies in whether to first extract the blood perfusion signal from the electrical impedance measurement signal, and then perform image reconstruction, or to first reconstruct the three-dimensional differential image based on the measurement signal, and then extract the special perfusion image from the three-dimensional differential image. The protection scope of the present disclosure is intended to include both implementations.

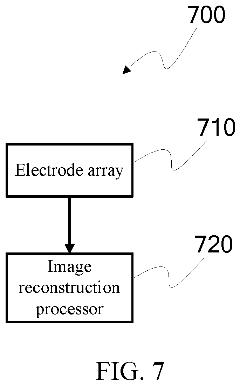

FIG. 7 is a schematic block diagram of an electrical impedance tomography based device 700 for generating a three-dimensional blood perfusion image according to an embodiment of the present disclosure.

As shown in FIG. 7, the electrical impedance tomography based device 700 for generating a three-dimensional blood perfusion image according to the embodiment of the present disclosure may include: an electrode array 710, distributed in a three-dimensional space, and configured to perform electrical impedance measurement on a human body region to be measured so as to obtain an electrical impedance measurement signal; and an image reconstruction processor 720, configured to execute a program stored in a memory, so as to reconstruct, based on a blood perfusion signal in an electrical impedance measurement signal, a three-dimensional blood perfusion image by means of an image reconstruction algorithm.

According to a preferred embodiment of the present disclosure, the electrode array 710 may be disposed on one or more impedance belts, an electrode vest, or an electrode cap, so as to realize three-dimensional distribution of electrodes.

According to a preferred implementation of the present disclosure, the image reconstruction processor 720 may be further configured to: execute a program stored in the memory, so as to extract the blood perfusion signal from the electrical impedance measurement signal; and reconstruct, by using the extracted blood perfusion signal, the three-dimensional blood perfusion image by means of the image reconstruction algorithm.

In an implementation, the image reconstruction processor 720 may be configured to extract the blood perfusion signal from the electrical impedance measurement signal by using a time-frequency characteristic of the signal. In an implementation, the image reconstruction processor 720 may be configured to separate, by a band-pass filter, a signal of a specific frequency range from the electrical impedance measurement signal to form the blood perfusion signal.

In a specific embodiment of the present disclosure, the image reconstruction algorithm is a linear differential imaging algorithm. However, those of ordinary skill in the art should understand that the image reconstruction algorithms that can be utilized in the present disclosure may include a variety of image reconstruction algorithms: linear or non-linear, iterative or non-iterative, random or deterministic image reconstruction algorithms, etc.

In addition, in another implementation of the present disclosure, the image reconstruction processor 720 may be further configured to: execute a program stored in the memory, so as to reconstruct a three-dimensional differential image by means of the image reconstruction algorithm; and extract the three-dimensional blood perfusion image reflected by the blood perfusion signal in the electrical impedance measurement signal from the three-dimensional differential image.

In an implementation, the image reconstruction processor 720 may use the same image reconstruction algorithm as in the first implementation when reconstructing the three-dimensional differential image. The image reconstruction processor 720 may be configured to extract the three-dimensional blood perfusion image by using a time-frequency characteristic of a pixel in the three-dimensional differential image. In an implementation, the image reconstruction processor 720 may be configured to separate, by a band-pass filter, the three-dimensional blood perfusion image from the three-dimensional differential image.

In addition, although not shown in FIG. 7, those skilled in the art should understand that, the device 700 may further include a display unit, such as a liquid crystal display (LCD), for displaying the reconstructed three-dimensional blood perfusion image. An example of the image is shown in FIG. 6. Since the present disclosure focuses more on the generation of the three-dimensional blood perfusion image, the display unit is not an essential element of the method or device of the present disclosure. The protection scope of the present disclosure is subjected to the claims, and is not limited to any embodiments described or not described in the present disclosure.

Furthermore, those of ordinary skill in the art should understand that the method of the present disclosure may be implemented by using a computer program. As described above in conjunction with FIGS. 1, 1A, and 1B, the method of the above embodiments may be implemented by using one or more programs, including instructing a computer or a processor to execute the algorithm shown in the drawings. The programs may be stored and provided to the computer or processor by using various types of non-transitory computer-readable media. These non-transitory computer-readable media include various types of tangible storage media. For example, the non-transitory computer-readable media may include magnetic recording media (such as floppy disks, magnetic tapes, and hard-disk divers), magneto-optical recording media (such as magneto-optical disks), compact disk read-only memories (CD-ROMs), compact disk-recordable (CD-R), compact disk re-writable (CD-R/W), and semiconductor memories (such as read-only memories (ROMs), programmable read-only memories (PROMs), erasable programmable read-only memories (EPROMs), flash read-only memories (ROMs), and random access memories (RAMs)). Further, the programs may be provided to the computer through various types of transitory computer-readable media. These transitory computer-readable media may include electrical signals, optical signals, and electromagnetic waves. The transitory computer-readable media may be configured to provide the programs to the computer through wired or wireless communication paths such as wires and optical fibers.

Therefore, according to the present disclosure, a computer program or a computer-readable medium for recording an instruction executable by a processor may further be proposed. When the instruction is executed by the processor, the processor implements an electrical impedance tomography based method for generating a three-dimensional blood perfusion image, which includes the following steps: performing, by using an electrode array distributed in a three-dimensional space, electrical impedance measurement on a human body region to be measured so as to obtain an electrical impedance measurement signal; and reconstructing, on the basis of a blood perfusion signal in the electrical impedance measurement signal, a three-dimensional blood perfusion image by means of an image reconstruction algorithm.

Various embodiments and implementations of the present disclosure have been described above, but the spirit and scope of the present disclosure are not limited thereto. Those skilled in the art may implement more applications according to the teachings of the present disclosure, but these applications are all within the scope of this disclosure.

In other words, the above-mentioned embodiments of the present disclosure are only examples for clearly illustrating the present disclosure, rather than limiting the implementations of the present disclosure. Those of ordinary skill in the art may make modifications or variations in other forms based on the above description. It is unnecessary and impossible to enumerate all the embodiments here. Any modifications, equivalent replacements, improvements, etc. made within the spirit and principle of the present disclosure should be included within the protection scope of the claims of the present disclosure.

## Claims

1. An electrical impedance tomography based method for generating a three-dimensional blood perfusion image, comprising:

   performing, by using an electrode array distributed in a three-dimensional space, electrical impedance measurement on a human body region to be measured so as to obtain an electrical impedance measurement signal; and
   reconstructing, on the basis of a blood perfusion signal in the electrical impedance measurement signal, a three-dimensional blood perfusion image by means of an image reconstruction algorithm.

2. The method according to claim 1, wherein the reconstructing, on the basis of a blood perfusion signal in the electrical impedance measurement signal, a three-dimensional blood perfusion image by means of an image reconstruction algorithm further comprises:

   extracting the blood perfusion signal from the electrical impedance measurement signal; and
   reconstructing, by using the extracted blood perfusion signal, the three-dimensional blood perfusion image by means of the image reconstruction algorithm.

3. The method according to claim 1, wherein the reconstructing, on the basis of a blood perfusion signal in the electrical impedance measurement signal, a three-dimensional blood perfusion image by an image reconstruction algorithm further comprises:

   reconstructing, on the basis of the electrical impedance measurement signal, a three-dimensional differential image by means of the image reconstruction algorithm; and
   extracting the three-dimensional blood perfusion image reflected by the blood perfusion signal in the electrical impedance measurement signal from the three-dimensional differential image.

4. The method according to claim 1, wherein the electrode array is disposed on one or more impedance bands, an electrode vest, or an electrode cap, so as to realize three-dimensional distribution of electrodes.

5. The method according to claim 2, wherein the extracting the blood perfusion signal from the electrical impedance measurement signal further comprises: extracting the blood perfusion signal from the electrical impedance measurement signal by using a time-frequency characteristic of the signal.

6. The method according to claim 5, wherein the extracting the blood perfusion signal from the electrical impedance measurement signal by using a time-frequency characteristic of the signal further comprises: separating, by a band-pass filter, a signal of a specific frequency range from the electrical impedance measurement signal to form the blood perfusion signal.

7. The method according to claim 3, wherein the extracting the three-dimensional blood perfusion image reflected by the blood perfusion signal in the electrical impedance measurement signal from the three-dimensional differential image further comprises: extracting the three-dimensional blood perfusion image by using a time-frequency characteristic of a pixel in the three-dimensional differential image.

8. The method according to claim 7, wherein the extracting the three-dimensional blood perfusion image by using a time-frequency characteristic of a pixel in the three-dimensional differential image further comprises: separating, by a band-pass filter, a signal of a specific frequency range from a time-domain signal of each pixel in the three-dimensional differential image, so as to form a time-domain signal of a corresponding pixel in the three-dimensional blood perfusion image.

9. An electrical impedance tomography based device for generating a three-dimensional blood perfusion image, comprising:

   an electrode array, distributed in a three-dimensional space, and configured to perform electrical impedance measurement on a human body region to be measured so as to obtain an electrical impedance measurement signal; and
   an image reconstruction processor, configured to execute a program stored in a memory, so as to reconstruct, on the basis of a blood perfusion signal in the electrical impedance measurement signal, a three-dimensional blood perfusion image by means of an image reconstruction algorithm.

10. The device according to claim 9, wherein the electrode array is disposed on one or more impedance bands, an electrode vest, or an electrode cap, so as to realize three-dimensional distribution of electrodes.

100

110

Performing, by using an electrode array distributed in a three-dimensional space, electrical impedance measurement on a human body region to be measured so as to obtain an electrical impedance measurement signal

120

Reconstructing, on the basis of a blood perfusion signal in the electrical impedance measurement signal, a three-dimensional blood perfusion image by means of an image reconstruction algorithm

FIG. 1

100A

110

> Performing, by using an electrode array distributed in a three-dimensional space, electrical impedance measurement on a human body region to be measured so as to obtain an electrical impedance measurement signal

121A

> Extracting a blood perfusion signal from the electrical impedance measurement signal

122A

> Reconstructing, by using the extracted blood perfusion signal, a three-dimensional blood perfusion image by means of an image reconstruction algorithm

FIG. 1A

100B

110

Performing, by using an electrode array distributed in a three-dimensional space, electrical impedance measurement on a human body region to be measured so as to obtain an electrical impedance measurement signal

121B

Reconstructing, on the basis of the electrical impedance measurement signal, a three-dimensional differential image by means of an image reconstruction algorithm

122B

Extracting a three-dimensional blood perfusion image reflected by a blood perfusion signal in the electrical impedance measurement signal from the three-dimensional differential image

FIG. 1B

（a）

（b）

FIG. 2

（a）

（b）

FIG. 3

（a）

（b）

（c）

FIG. 4

(a)

(b)

FIG. 5

FIG. 6

700

710

Electrode array

Image
reconstruction
processor

720

FIG. 7

## INTERNATIONAL SEARCH REPORT

| International application No. |
|---|
| **PCT/CN2020/106754** |

| **A.** | **CLASSIFICATION OF SUBJECT MATTER** |
|---|---|
| | A61B 5/053(2006.01)i; A61B 5/026(2006.01)i |

According to International Patent Classification (IPC) or to both national classification and IPC

| **B.** | **FIELDS SEARCHED** |
|---|---|

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNABS; CNTXT; VEN; USTXT; WOTXT; EPTXT; CNKI: 北京华睿博视, 张可, 张昕, 王谊冰, 于洋, 三维, 立体, 电阻抗, 血液, 灌注, impedance, three dimension+, stereoscop+, blood, perfus+

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | CN 111067521 A (BEIJING HUARUI BOSHI MEDICAL IMAGE TECHNOLOGY CO., LTD.) 28 April 2020 (2020-04-28)<br>claims 1-10 | 1-10 |
| X | CN 109864712 A (BEIJING HUARUI BOSHI MEDICAL IMAGE TECHNOLOGY CO., LTD.) 11 June 2019 (2019-06-11)<br>description, paragraphs [0009]-[0035], and figures 1-4 | 1-10 |
| X | CN 104582566 A (DRAEGER MEDICAL CO., LTD.) 29 April 2015 (2015-04-29)<br>description, paragraphs [0029]-[0032], figure 1 | 1-10 |
| A | CN 102688041 A (SEALAND TECHNOLOGY (CHENGDU) CO., LTD.) 26 September 2012 (2012-09-26)<br>entire document | 1-10 |
| A | CN 106264524 A (SPRING FOUNDATION OF NCTU) 04 January 2017 (2017-01-04)<br>entire document | 1-10 |
| A | WO 2018078540 A1 (NAVIX INTERNATIONAL LTD) 03 May 2018 (2018-05-03)<br>entire document | 1-10 |

☐ Further documents are listed in the continuation of Box C.  ☑ See patent family annex.

| | |
|---|---|
| \*   Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **14 September 2020** | **14 October 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT
### Information on patent family members

| International application No. |
|---|
| **PCT/CN2020/106754** |

| Patent document cited in search report | | | Publication date (day/month/year) | Patent family member(s) | | | Publication date (day/month/year) |
|---|---|---|---|---|---|---|---|
| CN | 111067521 | A | 28 April 2020 | None | | | |
| CN | 109864712 | A | 11 June 2019 | CN | 209847158 | U | 27 December 2019 |
| CN | 104582566 | A | 29 April 2015 | EP | 2884892 | A1 | 24 June 2015 |
| | | | | US | 2015216443 | A1 | 06 August 2015 |
| | | | | WO | 2014029631 | A1 | 27 February 2014 |
| | | | | DE | 102012214786 | A1 | 15 May 2014 |
| | | | | US | 10064568 | B2 | 04 September 2018 |
| | | | | BR | 112015003159 | A2 | 04 July 2017 |
| | | | | CN | 104582566 | B | 11 December 2018 |
| CN | 102688041 | A | 26 September 2012 | CN | 102688041 | B | 26 February 2014 |
| CN | 106264524 | A | 04 January 2017 | US | 2016341684 | A1 | 24 November 2016 |
| | | | | TW | I542324 | B | 21 July 2016 |
| | | | | US | 10422761 | B2 | 24 September 2019 |
| | | | | TW | 201641076 | A | 01 December 2016 |
| WO | 2018078540 | A1 | 03 May 2018 | US | 2020085504 | A1 | 19 March 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201911424085